# EUROPEAN PATENT APPLICATION

(11) **EP 3 141 185 A1**
(43) Date of publication of application: **15.03.2017**
(21) Application number: 16188509.0
(22) Date of filing: 13.09.2016
(51) Int. Cl.: A61B 5/00, A61B 5/042

(54) **CONVERTIBLE BASKET CATHETER**

(30) Priority: 14.09.2015 US 201514852727
(71) Applicant: Biosense Webster (Israel) Ltd., 2066717 Yokneam (IL)
(72) Inventor: BASU, Shubhayu, Irwindale, CA 91706 (US); AUJLA, Vishav Manak Singh, Irwindale, CA 91706 (US); WILLIAMS, Stuart, Irwindale, CA 91706 (US); SOLIS, Mario A., Irwindale, CA 91706 (US); AUERBACH, Shmuel, 2066717 Yokneam (IL)
(74) Representative: Carpmaels & Ransford LLP

(57) **Abstract**

This disclosure is directed to a catheter having an ellipsoidal basket-shaped electrode assembly at the distal end of the catheter body formed from a plurality of spines with electrodes. The ellipsoidal basket-shaped electrode assembly has a first deployed expanded configuration having a first area of electrode coverage and a first electrode density, a second deployed expanded configuration having a second area of electrode coverage less than the first area and a second electrode density higher than the first density, and a collapsed configuration wherein the spines are arranged generally along a longitudinal axis of the catheter body.

## Description

### FIELD OF THE PRESENT DISCLOSURE

This invention relates to electrophysiologic (EP) catheters, in particular, EP catheters for mapping and/or ablation in the heart.

### BACKGROUND

Electrophysiology catheters are commonly-used for mapping electrical activity in the heart. Various electrode designs are known for different purposes. In particular, catheters having basket-shaped electrode arrays are known and described, for example, in U.S. Patent Nos. 5,772,590, 6,748,255 and 6,973,340, the entire disclosures of each of which are incorporated herein by reference.

Basket catheters typically have an elongated catheter body and a basket-shaped electrode assembly mounted at the distal end of the catheter body. The basket assembly has proximal and distal ends and comprises a plurality of spines connected at their proximal and distal ends. Each spine comprises at least one electrode. The basket assembly has an expanded arrangement wherein the spines bow radially outwardly and a collapsed arrangement wherein the spines are arranged generally along the axis of the catheter body.

It is desirable that a basket assembly be capable of detecting in as few beats as possible, including a single beat, as much of the electrical function of the region in which the electrode assembly is deployed, such as the left or right atrium. Conventional basket-shaped electrode assemblies are generally spherical and may not provide an optimal conformation to the anatomy of the chamber in which they are deployed. Conventional basket-shaped electrode assemblies may also have difficulty presenting electrodes located in the polar areas adjacent the longitudinal axis of the catheter to the corresponding regions of the patient's heart. Still further, in some circumstances it may be desirable to obtain electrical signals from as wide an area as possible. However, in other circumstances, it may be desirable to convert the assembly to a different configuration in order to measure signals from a more localized area with a higher resolution. Conventional basket-shaped electrode assemblies are not convertible between different configurations with different measurement characteristics. Accordingly, the techniques of this disclosure as described in the following materials satisfy these and other needs.

### SUMMARY

The present disclosure is directed to a catheter with an elongated catheter body having proximal and distal ends and at least one lumen therethrough and an ellipsoidal basket-shaped electrode assembly at the distal end of the catheter body, the ellipsoidal basket-shaped electrode assembly comprising a plurality of spines connected at their proximal and distal ends, each spine comprising a plurality of electrodes, wherein the ellipsoidal basket-shaped electrode assembly has a first deployed expanded configuration having a first area of electrode coverage and a first electrode density, a second deployed expanded configuration having a second area of electrode coverage less than the first area and a second electrode density higher than the first density, and a collapsed configuration wherein the spines are arranged generally along a longitudinal axis of the catheter body.

In one aspect, the spines bow radially outwardly in the first deployed expanded configuration.

In one aspect, each spine loops back on itself in the second deployed expanded configuration.

In one aspect, the ellipsoidal basket-shaped electrode assembly may have a longitudinal axis length that is shorter than an equatorial axis length when in the second deployed expanded configuration

In one aspect, the catheter may also include an puller having proximal and distal ends, the puller slidably disposed within the lumen and aligned with the longitudinal axis of the catheter body, wherein the plurality of spines are attached at their distal ends to the puller, such that the ellipsoidal basket-shaped electrode assembly has the collapsed configuration when the puller is at a most distal position along the longitudinal axis relative to the catheter body. Proximal movement of the puller through a first range of travel may be associated with conversion of the ellipsoidal basket-shaped electrode assembly to the first deployed expanded configuration from the collapsed configuration. Further proximal movement of the puller through a second range of travel converts the ellipsoidal basket-shaped electrode assembly to the second deployed expanded configuration from the first deployed expanded configuration. The catheter may also have a cap to secure the distal ends of each spine, wherein the puller is attached to the cap and movement of the puller through the second range of travel brings the cap adjacent the distal end of the catheter body.

In one aspect, the ellipsoidal basket-shaped electrode assembly may have a longitudinal length at least equal to an equatorial length when in the first deployed expanded configuration.

In one aspect, each spine may have a concave distal region, a convex middle region and a concave proximal region when in the second deployed expanded configuration. The convex middle region may have a middle area of flattened curvature. Alternatively or in addition, the convex middle region may have proximal and/or distal areas of flattened curvature.

This disclosure is also directed to a method for mapping a chamber of a heart by providing a catheter having an elongated catheter body with proximal and distal ends and at least one lumen therethrough and an ellipsoidal basket-shaped electrode assembly at the distal end of the catheter body, the ellipsoidal basket-shaped electrode assembly comprising a plurality of spines connected at their proximal and distal ends, each spine comprising a plurality of electrodes. The distal end of the catheter may be introduced into the chamber, the ellipsoidal basket-shaped electrode assembly may be expanded from a collapsed configuration wherein the spines are arranged generally along a longitudinal axis of the catheter body to a first deployed expanded configuration having a first area of electrode coverage and a first electrode density, the ellipsoidal basket-shaped electrode assembly may be converted from the first deployed expanded configuration to a second deployed expanded configuration having a second area of electrode coverage less than the first area and a second electrode density higher than the first density, the ellipsoidal basket-shaped electrode assembly may then be positioned within the chamber so that at least a portion of the electrodes are in contact with tissue forming the chamber and electrical data received from the at least a portion of the electrodes in contact with the tissue may be recorded.

In one aspect, the chamber of the heart may be an atrium or a ventricle. Positioning the ellipsoidal basket-shaped electrode assembly within the chamber may include manipulating the catheter so that the second deployed expanded configuration of the ellipsoidal basket-shaped electrode assembly abuts an atrial wall.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
FIG. 1 is a top plan view of a catheter of the present invention, according to one embodiment.
FIG. 2 is a schematic view of an ellipsoidal basket-shaped electrode assembly in a first deployed expanded configuration, according to one embodiment.
FIG. 3 is a schematic view of one spine of the ellipsoidal basket-shaped electrode assembly of FIG. 2.
FIG. 4A is an elevational view of an ellipsoidal basket-shaped electrode assembly in a second deployed expanded configuration, according to one embodiment.
FIG. 4B is a side view of an ellipsoidal basket-shaped electrode assembly in a second deployed expanded configuration, according to one embodiment.
FIG. 4C is a top view of an ellipsoidal basket-shaped electrode assembly in a second deployed expanded configuration, according to one embodiment.
FIG. 5 is a schematic view of an ellipsoidal basket-shaped electrode assembly in a first deployed expanded configuration within the left atrium, according to one embodiment.
FIG. 6 is a schematic view of an ellipsoidal basket-shaped electrode assembly in a second deployed expanded configuration within the left atrium, according to one embodiment.
FIG. 7 is another schematic view of an ellipsoidal basket-shaped electrode assembly in a second deployed expanded configuration within the left atrium, according to one embodiment.
FIG. 8 is a schematic illustration of an invasive medical procedure using an ellipsoidal basket-shaped electrode assembly, according to one embodiment.

### DETAILED DESCRIPTION

At the outset, it is to be understood that this disclosure is not limited to particularly exemplified materials, architectures, routines, methods or structures as such may vary. Thus, although a number of such options, similar or equivalent to those described herein, can be used in the practice or embodiments of this disclosure, the preferred materials and methods are described herein.

It is also to be understood that the terminology used herein is for the purpose of describing particular embodiments of this disclosure only and is not intended to be limiting.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details. In some instances, well known structures and devices are shown in block diagram form in order to avoid obscuring the novelty of the exemplary embodiments presented herein.

For purposes of convenience and clarity only, directional terms, such as top, bottom, left, right, up, down, over, above, below, beneath, rear, back, and front, may be used with respect to the accompanying drawings. These and similar directional terms should not be construed to limit the scope of the disclosure in any manner.

Unless defined otherwise, all technical and scientific terms used herein have the same meaning as commonly understood by one having ordinary skill in the art to which the disclosure pertains.

Finally, as used in this specification and the appended claims, the singular forms "a, "an" and "the" include plural referents unless the content clearly dictates otherwise.

Certain types of electrical activity within a heart chamber are not cyclical. Examples include arterial flutter or arterial fibrillation, and ventricular tachycardia originating in scars in the wall of the ventricle that have resulted from infarcts. Such electrical activity is random from beat to beat. To analyze or 'map' this type of electrical activity, it is desirable to obtain the 'picture' as quickly as possible, such as within one heartbeat. In other words, all the points of the map or picture may be obtained simultaneously within one-tenth of a second. According to the techniques of this disclosure, an ellipsoidal basket-shaped electrode assembly may conform more closely to the anatomy of the patient's heart in order to accurately map this electrical activity. Further, the disclosed basket-shaped electrode assembly is also convertible between a first deployed expanded configuration capable of measuring electrical signals across a first area at a first resolution and a second deployed expanded configuration capable of measuring electrical signals across a second, more localized area at a second, increased resolution.

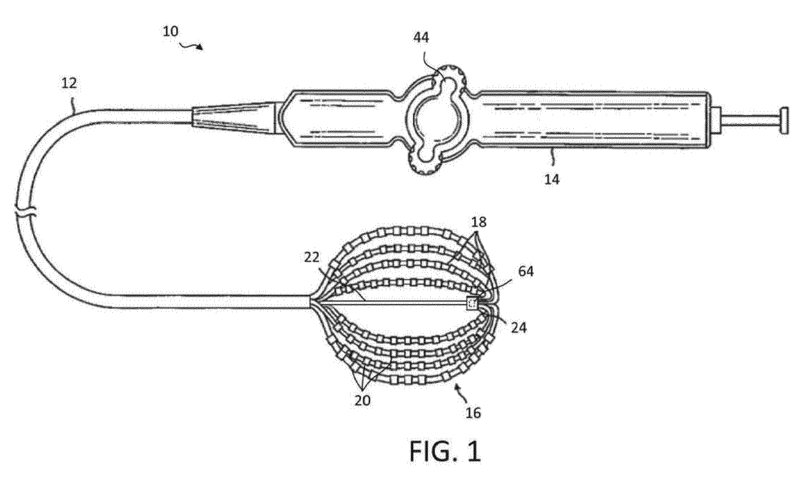

As shown in FIG. 1, the catheter 10 comprises an elongated catheter body 12 having proximal and distal ends and a control handle 14 at the proximal end of the catheter body, with a basket-shaped electrode assembly 16 having a plurality of spines 18, each carrying multiple electrodes 20, mounted at the distal end of the catheter body 12. The catheter body 12 comprises an elongated tubular construction having a single, axial or central lumen (not shown), but can optionally have multiple lumens if desired. To enable accurate mapping of electrical signals, for example to detect most or substantially all of the electrical function of the right or left atrium in as little as a single heartbeat, it may be desirable to provide an array of electrodes with a relatively high density. As such, numbers of spines 18 employed may be eight, ten, twelve or any other suitable number. Spines 18 may be evenly or unevenly distributed radially. Further, each spine 18 may include multiple electrodes 20, such as at least ten and up to approximately 16 electrodes per spine. Similarly, the electrodes may be evenly distributed along the spine or may be skewed proximally, centrally or distally to facilitate analysis of the measured electrical signals.

The catheter body 12 is flexible, i.e., bendable, but substantially non-compressible along its length. The catheter body 12 can be of any suitable construction and made of any suitable material. One construction comprises an outer wall made of polyurethane or PEBAX® (polyether block amide). The outer wall comprises an imbedded braided mesh of stainless steel or the like to increase torsional stiffness of the catheter body 12 so that, when the control handle 14 is rotated, the distal end of the catheter body will rotate in a corresponding manner. The outer diameter of the catheter body 12 is not critical, but generally should be as small as possible and may be no more than about 10 french depending on the desired application. Likewise the thickness of the outer wall is not critical, but may be thin enough so that the central lumen can accommodate a puller wire, lead wires, sensor cables and any other wires, cables or tubes. If desired, the inner surface of the outer wall is lined with a stiffening tube (not shown) to provide improved torsional stability. An example of a catheter body construction suitable for use in connection with the present invention is described and depicted in U.S. Patent No. 6,064,905, the entire disclosure of which is incorporated herein by reference.

The basket-shaped electrode assembly 16 may also include a puller 22 is generally coaxial with the catheter body 12 and extends from the proximal end of catheter body 12 through the central lumen and is attached, directly or indirectly, to the distal ends of spines 18. The puller 22 is afforded longitudinal movement relative to the catheter body so that it can move the distal ends of the spines 18 proximally or distally relative to the catheter body 12 to radially expand and contract, respectively, the electrode assembly. Since the proximal ends of spines 18 are secured to the catheter body 12, the distance between the distal and proximal ends of spines 18 shortens when they bow outwards into an expanded arrangement, which may be associated with relative movement of puller 22 in the proximal direction. Alternatively or in addition, spines 18 may include a material as described below that facilitates assuming the expanded arrangement, such as a shape memory material, so that puller 22 may be omitted or may be used to aid the transition between the expanded and collapsed arrangements. In an embodiment, the puller 22 may comprise a wire or hypotube formed from a suitable shape memory material, such as a nickel titanium alloy as described below. As will be appreciated, different relative amounts of movement of the puller 22 along the longitudinal axis may affect the degree of bowing, such as to enable the spines 18 to exert greater pressure on the atrial tissue for better contact between the tissue and the electrodes on the spines. Thus, a user can modify the shape of the electrode assembly by adjusting the longitudinal extension or withdrawal of the puller.

A first range of travel of puller 22 from its most distal location to a relatively more proximal location corresponds to deflection of basket-shaped electrode assembly 16 from a collapsed configuration to a first deployed expanded configuration having the generally ellipsoidal shape shown in FIG. 1. When in the collapsed configuration, the spines may be constrained, such as by a guiding sheath. Further, spines 18 may include a sufficient resilient material so that they assume the first expanded deployed configuration when unconstrained with relatively little or no force applied to puller 22. Alternatively, spines 18 may be configured to remain in the collapsed configuration even when unconstrained so that they may be deflected from the collapsed configuration to the first expanded deployed configuration by imparting sufficient force to puller 22. As will be appreciated, in the collapsed configuration, spines 18 assume a generally linear alignment with the catheter body 12 to minimize the outer diameter for insertion within and withdrawal from the patient. In expanding to the first deployed expanded configuration, spines 18 of basket-shaped electrode assembly 16 bow outwards. When positioned at a desired location within a patient, assuming the first deployed expanded configuration may bring electrodes 20 into contract or closer proximity with the walls of the chamber. In one aspect, the ellipsoidal shape of basket-shaped electrode assembly 16 in the first deployed expanded configuration may be characterized as having a length along its longitudinal axis that is at least equal to a length along its equatorial axis. Further, in some embodiments the longitudinal axis length is greater than the equatorial axis length, so that the longitudinal axis length to the equatorial axis length may have a ratio in the range of 5-9:5-8, such as the ratios of 5.5:5, 6.5:6 and 7:6 which are exemplary only and not limiting. In one embodiment, basket-shaped electrode assembly 16 may have a length of approximately 65 mm and a width of approximately 55 mm when in the first deployed expanded configuration. Different ratios and sizes may be employed depending on the patient's anatomy to provide a close fit to the area of the patient being investigated, such as the right or left atria.

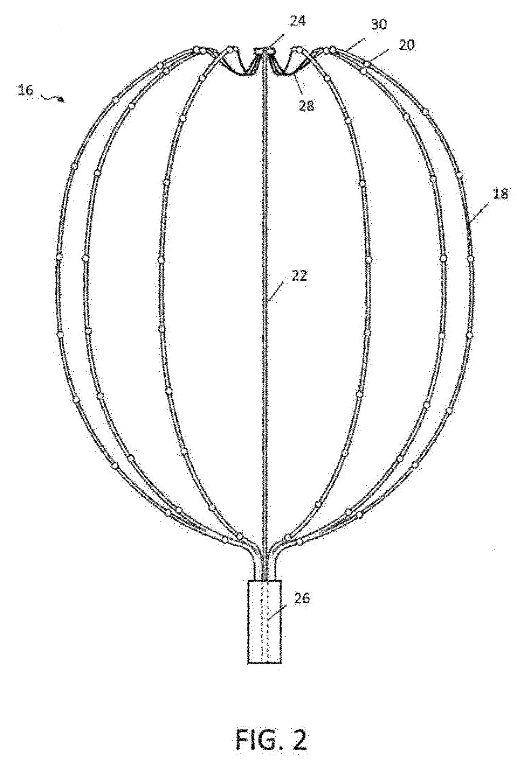

A detailed view of one embodiment of the basket-shaped electrode assembly 16 is shown in FIG. 2, showing six spines 18, each carrying ten electrodes 20, of a ten total spine configuration (the middle four spines are omitted in this view to improve clarity). As noted above, in other embodiments, different numbers of spines 18 and/or electrodes 20 may be employed, each of which may be evenly or unevenly distributed as desired. The distal ends of the spines 18 and the puller 22 may be secured to a distal cap 24. Correspondingly, the proximal ends of the spines 18 may be secured to the distal end of the catheter body 12, while the puller 22 may be routed through lumen 26 of the catheter body 12 so that the proximal end extends to the control handle 14. In some embodiments, lumen 26 may also be used to supply a suitable irrigation fluid, such as heparinized saline, to the basket-shaped electrode assembly 16. A fitting (not shown) in the control handle 14 may be provided to conduct irrigation fluid from a suitable source or pump into the lumen 26.

Each spine 18 may comprise a flexible wire 28 with a non-conductive covering 30 on which one or more of the ring electrodes 20 are mounted. In an embodiment, the flexible wires 28 may be formed from a shape memory material to facilitate the transition between expanded and collapsed arrangements and the non-conductive coverings 30 may each comprise a biocompatible plastic tubing, such as polyurethane or polyimide tubing. For example, nickel-titanium alloys known as nitinol may be used. At body temperature, nitinol wire is flexible and elastic and, like most metals, nitinol wires deform when subjected to minimal force and return to their shape in the absence of that force. Nitinol belongs to a class of materials called Shaped Memory Alloys (SMA) that have interesting mechanical properties beyond flexibility and elasticity, including shape memory and superelasticity which allow nitinol to have a "memorized shape" that is dependent on its temperature phases. The austenite phase is nitinol's stronger, higher-temperature phase, with a simple cubic crystalline structure. Superelastic behavior occurs in this phase (over a 50°-60°C temperature spread). Correspondingly, the martensite phase is a relatively weaker, lower-temperature phase with a twinned crystalline structure. When a nitinol material is in the martensite phase, it is relatively easily deformed and will remain deformed. However, when heated above its austenite transition temperature, the nitinol material will return to its pre-deformed shape, producing the "shape memory" effect. The temperature at which nitinol starts to transform to austenite upon heating is referred to as the "As" temperature. The temperature at which nitinol has finished transforming to austenite upon heating is referred to as the "Af" temperature. Accordingly, the basket-shaped electrode assembly 16 may have a three dimensional shape that can be easily collapsed to be fed into a guiding sheath and then readily returned to its expanded shape memory configuration upon delivery to the desired region of the patient upon removal of the guiding sheath.

Alternatively, in some embodiments the spines 18 can be designed without the internal flexible wire 28 if a sufficiently rigid nonconductive material is used for the non-conductive covering 30 to permit radial expansion of the basket-shaped electrode assembly 16, so long as the spine has an outer surface that is non-conductive over at least a part of its surface for mounting of the ring electrodes 20.

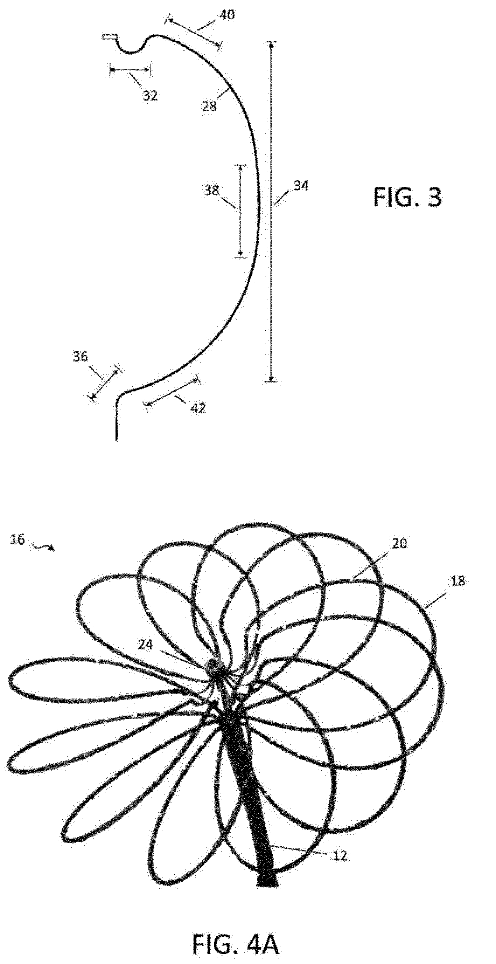

The internal flexible wire 28 of a single spine 18 is shown in its expanded, first deployed expanded configuration in FIG. 3. In this embodiment, wire 28 has a distal region 32 exhibiting a concave configuration, positioned generally within a radius of curvature defined by the adjacent proximal portion. This invaginated design of distal region 32 keeps the top of basket-shaped electrode assembly 16 flush with the outer curvature for safety reasons, by presenting a blunter and atraumatic surface. Further, an electrode 20 may be positioned at the inflexion point where concave distal region 32 transitions to a convex middle region 34. A proximal region 36 may have a concave configuration, positioned generally outside the radius of curvature defined by the adjacent distal portion. This configuration provides a smooth transition from the middle region 34 to the flexible wire again being in alignment with the longitudinal axis.

As desired, middle region 34 may exhibit varying degrees of curvature in order to more closely conform to the anatomy of the patient. For example, a relatively flattened area 38 of middle region 34 may be configured to provide enhanced contact and/or positioning relative to the roof and floor of the atrium, while a relatively flattened area 40 at the distal end and a relatively flattened area 42 at the proximal end may provide better electrode contact with the lateral and septal walls, respectively. Other radii of curvature or similar conformational modifications may be made to adapt to the area in which basket-shaped electrode assembly 16 is intended to be deployed.

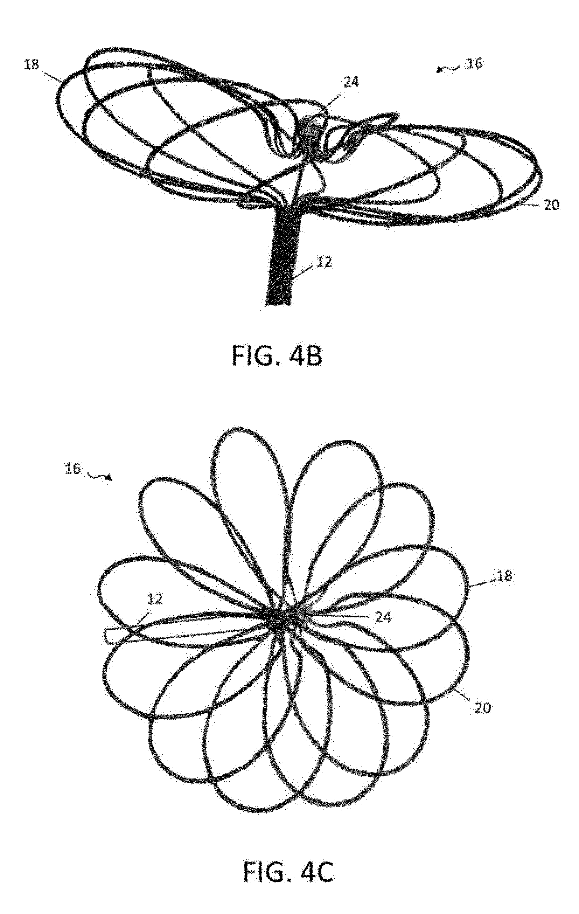

Basket-shaped electrode assembly 16 may also exhibit a second deployed expanded configuration as noted above. Puller 22 may undergo a second range of travel from the relatively more proximal location associated with the end of the first range of travel to approach a completely proximal location that causes each spine 18 to loop back upon itself as shown in FIGs. 4A-C. Generally, the second deployed expanded configuration may be characterized by having a longitudinal axis length that is shorter than the equatorial axis length. In some embodiments, the longitudinal axis length to the equatorial axis length has a ratio of 1:2 or less and in one example, the distal end of each spine may be substantially adjacent to the proximal end when in the second deployed expanded configuration. A three-quarters elevational view of the second deployed expanded configuration is shown in FIG. 4A, while FIG. 4B illustrates a top view and FIG. 4C illustrates a side view. Puller 22 has been moved proximally until distal cap 24 abuts the distal end of catheter body 12. The total area that may be accessed using electrodes 20 when basket-shaped electrode assembly 16 is in the second deployed expanded configuration is reduced relative to the first deployed expanded configuration. Correspondingly, the density of electrodes in the second deployed expanded configuration is increased relative to the first deployed expanded configuration, since the same number of electrodes is present. For example, the second deployed expanded configuration may have an electrode density approximately twice that of the first deployed expanded configuration. By positioning puller 22 in varying locations, basket-shaped electrode assembly 16 may selectively take on the first or the second deployed expanded configuration, or any configuration between. As such, this can be seen to offer the potential to perform global or regional interrogations using a single catheter design.

In some embodiments, puller 22 may be coupled to an actuator 44 on control handle 14 as shown in FIG. 1. Actuator 44 may be a sliding lever, a rotating knob or any other suitable implementation. As such, actuator 44 may be used to adjust the relative longitudinal position of puller 22 and in particular may be configured adjust the position of puller 22 at least through the second range of travel. Further, actuator 44 may be configured to hold puller 22 in an adjusted position that corresponds to a desired configuration of ellipsoidal basket-shaped electrode assembly 16, such as the first deployed expanded configuration and/or the second deployed expanded configuration as well as intermediate positions if desired.

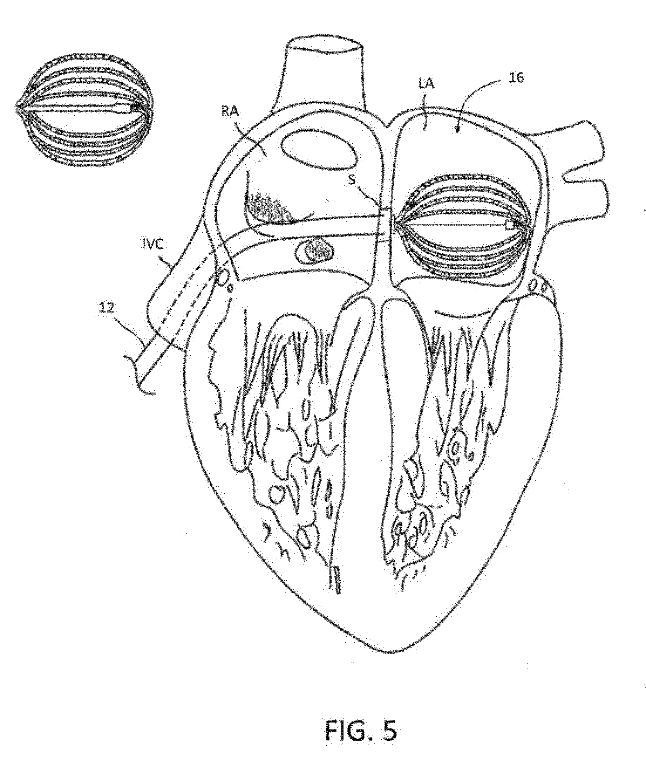

In one aspect, an electrophysiologist may introduce a guiding sheath, guidewire and dilator into the patient, as is generally known in the art. Examples of suitable guiding sheaths for use in connection with the inventive catheter are the PREFACE™ Braided Guiding Sheath (commercially available from Biosense Webster, Inc., Diamond Bar, CA) and the DiRex™ Guiding Sheath (commercially available from BARD, Murray Hill, NJ). The guidewire is inserted, the dilator is removed, and the catheter is introduced through the guiding sheath whereby the guidewire lumen in the puller permits the catheter to pass over the guidewire. In one exemplary procedure as depicted in FIG. 5, the catheter is first introduced to the right atrium (RA) via the inferior vena cava (IVC), where it passes through the septum (S) in order to reach the left atrium (LA).

As will be appreciated, the guiding sheath covers the spines 18 of the basket-shaped electrode assembly 16 in a collapsed position so that the entire catheter can be passed through the patient's vasculature to the desired location. The puller 22 may be positioned distally of the catheter body to allow the spines of the assembly to be flattened while the assembly is passed through the guiding sheath. Once the distal end of the catheter reaches the desired location, e.g., the left atrium, the guiding sheath is withdrawn to expose the basket-shaped electrode assembly 16. The puller 22 is drawn proximally through its first range of travel or otherwise manipulated so that the spines 18 flex outwardly between the distal and proximal junctions. With the basket-shaped electrode assembly 16 radially expanded, the ring electrodes 20 contact atrial tissue. As recognized by one skilled in the art, the basket-shaped electrode assembly 16 may be fully or partially expanded into the first deployed expanded configuration as shown in FIG. 5. Further, aspects of the configuration of basket-shaped electrode assembly 16 may be tailored to more closely conform to the area in which it is deployed as discussed above. In this embodiment, flattened area 38 of middle region 34 as shown in FIG. 3 may be configured to provide enhanced contact and/or positioning relative to the floor(or roof) of the atrium. Likewise, the relatively flattened areas 40 and 42 as shown in FIG. 3 may provide better electrode contact with the lateral and septal walls, respectively.

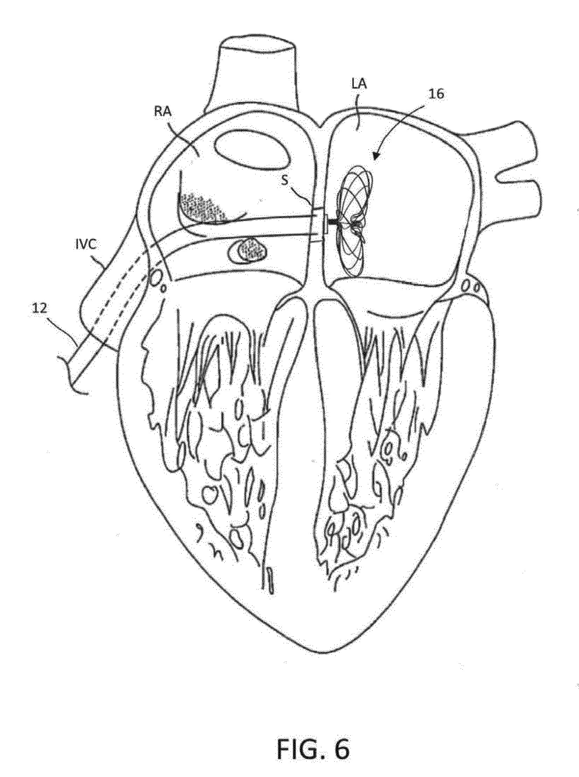

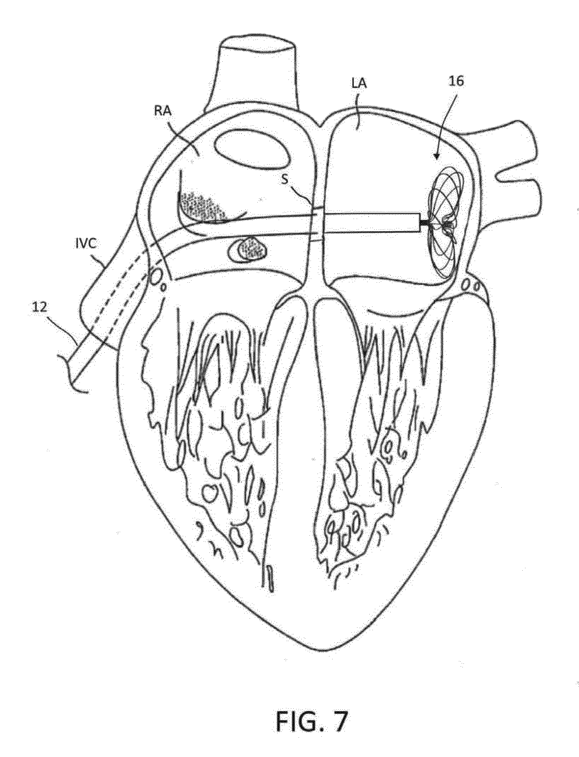

Alternatively or in addition, puller 22 may be drawn proximally through the second range of travel or otherwise manipulated so that the spines 18 loop back on themselves to assume the second deployed expanded configuration. As discussed above, the second deployed expanded configuration represents a reduced area of electrode coverage with a higher density. In one aspect, basket-shaped electrode assembly 16 when in the second deployed expanded configuration may be drawn back against septal tissue as shown in FIG. 6 to map this area with a higher electrode density. Similarly, basket-shaped electrode assembly 16 may also be pushed forward against the atrial free wall as shown in FIG. 7.

When the basket-shaped electrode assembly 16 is expanded into either the first deployed expanded configuration or the second deployed expanded configuration, the electrophysiologist may map local activation time and/or ablate using electrodes 20, which can guide the electrophysiologist in diagnosing and providing therapy to the patient. The catheter may include one or more reference ring electrodes mounted on the catheter body and/or one or more reference electrodes may be placed outside the body of the patient. By using the inventive catheter with the multiple electrodes on the basket-shaped electrode assembly, the electrophysiologist can obtain a true anatomy of a cavernous region of the heart, including an atrium, by measuring less points than with traditional catheters, allowing a more rapid mapping of the region.

In a further aspect, each spine 18 may include cabling with built-in or embedded lead wires for the electrodes 20 carried by the spine as described in U.S. Patent Publication No. 2014/0309512, published October 16, 2014, entitled HIGH DENSITY ELECTRODE STRUCTURE, and U.S. Patent Publication No. 2014/0305699, published October 16, 2014, entitled CONNECTION OF ELECTRODES TO WIRES COILED ON A CORE, the entire disclosures of which are hereby incorporated by reference.

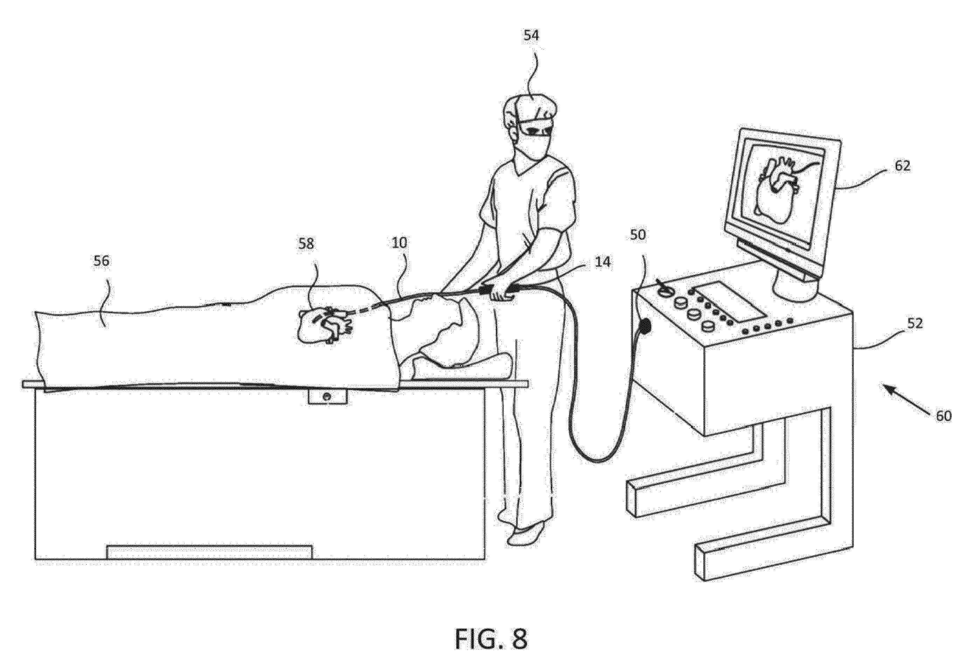

To help illustrate use of the basket-shaped electrode assembly 16, FIG. 8 is a schematic depiction of an invasive medical procedure, according to an embodiment of the present invention. Catheter 10, with the basket-shaped electrode assembly 16 (not shown in this view) at the distal end may have a connector 50 at the proximal end for coupling the wires from their respective electrodes 20 (not shown in this view) to a console 52 for recording and analyzing the signals they detect. An electrophysiologist 54 may insert the catheter 10 into a patient 56 in order to acquire electropotential signals from the heart 58 of the patient. The professional uses the control handle 14 attached to the catheter in order to perform the insertion. Console 52 may include a processing unit 60 which analyzes the received signals, and which may present results of the analysis on a display 62 attached to the console. The results are typically in the form of a map, numerical displays, and/or graphs derived from the signals.

In a further aspect, the processing unit 60 may also receive signals from one or more location sensors 64 provided near a distal end of the catheter 10 adjacent the basket-shaped electrode assembly 16 as schematically indicated in FIG. 1. The sensor(s) may each comprise a magnetic-field-responsive coil or a plurality of such coils. Using a plurality of coils enables six-dimensional position and orientation coordinates to be determined. The sensors may therefore generate electrical position signals in response to the magnetic fields from external coils, thereby enabling processor 60 to determine the position, (e.g., the location and orientation) of the distal end of catheter 10 within the heart cavity. The electrophysiologist may then view the position of the basket-shaped electrode assembly 16 on an image the patient's heart on the display 62. By way of example, this method of position sensing may be implemented using the CARTO™ system, produced by Biosense Webster Inc. (Diamond Bar, Calif.) and is described in detail in U.S. Patent Nos. 5,391,199, 6,690,963; 6,484,118; 6,239,724; 6,618,612 and 6,332,089, in PCT Patent Publication WO 96/05768, and in U.S. Patent Application Publication Nos. 2002/0065455 A1, 2003/0120150 A1 and 2004/0068178 A1, whose disclosures are all incorporated herein by reference. As will be appreciated, other location sensing techniques may also be employed. If desired, at least two location sensors may be positioned proximally and distally of the basket-shaped electrode assembly 16. The coordinates of the distal sensor relative to the proximal sensor may be determined and, with other known information pertaining to the curvature of the spines 18 of the basket-shaped electrode assembly 16, used to find the positions of each of the electrodes 20.

The preceding description has been presented with reference to presently disclosed embodiments of the invention. Workers skilled in the art and technology to which this invention pertains will appreciate that alterations and changes in the described structure may be practiced without meaningfully departing from the principal, spirit and scope of this invention. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A catheter comprising an elongated catheter body having proximal and distal ends and at least one lumen therethrough and an ellipsoidal basket-shaped electrode assembly at the distal end of the catheter body, the ellipsoidal basket-shaped electrode assembly comprising a plurality of spines connected at their proximal and distal ends, each spine comprising a plurality of electrodes, wherein the ellipsoidal basket-shaped electrode assembly has a first deployed expanded configuration having a first area of electrode coverage and a first electrode density, a second deployed expanded configuration having a second area of electrode coverage less than the first area and a second electrode density higher than the first density, and a collapsed configuration wherein the spines are arranged generally along a longitudinal axis of the catheter body.

2. The catheter of claim 1, wherein the spines bow radially outwardly in the first deployed expanded configuration.

3. The catheter of claim 1, wherein each spine loops back on itself in the second deployed expanded configuration.

4. The catheter of claim 3, wherein the ellipsoidal basket-shaped electrode assembly has a longitudinal axis length that is shorter than an equatorial axis length when in the second deployed expanded configuration.

5. The catheter of claim 1, further comprising an puller having proximal and distal ends, the puller slidably disposed within the lumen and aligned with the longitudinal axis of the catheter body, wherein the plurality of spines are attached at their distal ends to the puller, such that the ellipsoidal basket-shaped electrode assembly has the collapsed configuration when the puller is at a most distal position along the longitudinal axis relative to the catheter body.

6. The catheter of claim 5, wherein proximal movement of the puller through a first range of travel is associated with conversion of the ellipsoidal basket-shaped electrode assembly to the first deployed expanded configuration from the collapsed configuration.

7. The catheter of claim 6, wherein further proximal movement of the puller through a second range of travel converts the ellipsoidal basket-shaped electrode assembly to the second deployed expanded configuration from the first deployed expanded configuration.

8. The catheter of claim 7, further comprising a cap securing the distal ends of each spine, wherein the puller is attached to the cap and movement of the puller through the second range of travel brings the cap adjacent the distal end of the catheter body.

9. The catheter of claim 1, wherein the ellipsoidal basket-shaped electrode assembly has a longitudinal length at least equal to an equatorial length when in the first deployed expanded configuration.

10. The catheter of claim 1, wherein each spine has a concave distal region, a convex middle region and a concave proximal region when in the first deployed expanded configuration.

11. The catheter of claim 10, wherein the convex middle region has a middle area of flattened curvature.

12. The catheter of claim 11, wherein the convex middle region has proximal and distal areas of flattened curvature.

13. A method for mapping a chamber of a heart comprising:
providing a catheter having an elongated catheter body with proximal and distal ends and at least one lumen therethrough and an ellipsoidal basket-shaped electrode assembly at the distal end of the catheter body, the ellipsoidal basket-shaped electrode assembly comprising a plurality of spines connected at their proximal and distal ends, each spine comprising a plurality of electrodes,
introducing the distal end of the catheter into the chamber;
expanding the ellipsoidal basket-shaped electrode assembly from a collapsed configuration wherein the spines are arranged generally along a longitudinal axis of the catheter body to a first deployed expanded configuration having a first area of electrode coverage and a first electrode density;
converting the ellipsoidal basket-shaped electrode assembly from the first deployed expanded configuration to a second deployed expanded configuration having a second area of electrode coverage less than the first area and a second electrode density higher than the first density; and
positioning the ellipsoidal basket-shaped electrode assembly within the chamber so that at least a portion of the electrodes are in contact with tissue forming the chamber; and
recording electrical data received from the at least a portion of the electrodes in contact with the tissue.

14. The method of claim 13, wherein the chamber of the heart is an atrium or a ventricle.

15. The method of claim 14, wherein positioning the ellipsoidal basket-shaped electrode assembly within the chamber comprises manipulating the catheter so that the second deployed expanded configuration of the ellipsoidal basket-shaped electrode assembly abuts an atrial wall.
second deployed expanded configuration
